Europäisches Patentamt

European Patent Office

Office européen des brevets

(11) Publication number: **0 412 746 A2**

# EUROPEAN PATENT APPLICATION

(21) Application number: 90308614.8

(51) Int. Cl.5: **G01N 27/06**

(22) Date of filing: 06.08.90

(30) Priority: 08.08.89 US 391148
29.03.90 US 503518

(43) Date of publication of application:
13.02.91 Bulletin 91/07

(84) Designated Contracting States:
AT BE CH DE DK ES FR GB GR IT LI LU NL SE

(71) Applicant: **Klein, Lawrence W.**
**Rural Route 1, Box 20A**
**Alhambra, Illinois 62001(US)**

(72) Inventor: **Klein, Lawrence W.**
**Rural Route 1, Box 20A**
**Alhambra, Illinois 62001(US)**

(74) Representative: **Simpson, Ronald Duncan**
**Innes et al**
**A.A.Thornton & Co. Northumberland House**
**303-306 High Holborn**
**London WCIV 7LE(GB)**

(54) **Fluid testing device and method.**

(57) A fluid tester for measuring the quality of a fluid such as brake fluid or coolant, comprising means for measuring the conductivity of the fluid, preferably comprising a plurality of comparators for comparing the resistance of the fluid to a plurality of reference resistances. The device further comprises indicating means, preferably light emitting diodes, responsive to the measuring means for indicating the quality of the fluid based upon its measured conductivity. An increasing number of diodes are illuminated as the conductivity of the fluid increases, providing a positive indication when the device is working and providing an easy to interpret display of the test results. The diodes are preferably activated at preselected levels of conductivity, corresponding to preselected levels of contamination. In one embodiment, the measuring means comprises a probe having first and second terminals adapted to be in contact with the fluid, means for switching the polarity of the first and second terminals and means for repetitively actuating the switching means whereby the first and second terminals successively change polarity to inhibit ionization of the fluid between the first and second terminals. A method of testing is also disclosed.

EP 0 412 746 A2

## FLUID TESTING DEVICE AND METHOD

BACKGROUND OF THE INVENTION

This invention relates to a device for testing fluid, and in particular to a device for testing the quality of brake fluid and/or coolant by measuring its conductivity.

Water and corrosion products contaminate brake fluids, adversely affecting their performance and reducing their boiling points. Brake fluids are generally hygroscopic, i.e., they readily absorb water, and thus they rapidly deteriorate when exposed to water. This is a problem even for fresh brake fluid if it is not properly sealed. It has been experimentally determined that one percent moisture will lower the boiling point as much as 25% and three percent moisture will lower the boiling point 50%. This decrease in boiling point means that heat generated during the operation of the brakes can cause the water to boil out of the brake fluid, forming steam that impairs the operation of the brakes.

The deterioration of brake fluid is usually not apparent from the appearance of the brake fluid. For this reason at least some automobile manufacturers recommend that the brake fluid be frequently changed. Prior testing equipment for brake fluid was complicated, time consuming, and expensive. One prior device for testing brake fluid is disclosed in Klein et al., U.S. Patent No. 4,566,805. This device operates by monitoring the temperature of a small heating element immersed in the fluid as the element is heated. The temperature of the element levels off briefly at the boiling point of the fluid, indicating the moisture content of the fluid.

Water and corrosion products also contaminate coolants, adversely affecting their performance. These contaminants set up electrolytic cells which result in electrolysis causing corrosion and severely reducing the useful life of component parts of automotive cooling system. The contaminants also decrease the boiling point of the coolant which means that heat generated during the operation of the automobile can cause the water to boil out of the coolant, forming steam that impairs the operation of the cooling system of the automobile.

The deterioration of coolants is usually not apparent from the appearance of the coolant. For this reason at least some automobile manufacturers recommend that the coolant be frequently changed.

SUMMARY OF THE INVENTION

It is among the objects of the present invention to provide a device for testing the quality of fluids such as brake fluids and coolants, and in particular to provide such a device that tests the quality of brake fluid and coolant by measuring its conductivity or resistance and comparing it to preselected bench marks. It is also among the objects of the present invention to provide such a device that measures the conductivity by comparing it to a reference. It is also among the objects of the present invention to provide such a device that is of simple and inexpensive construction; to provide such a device that provides a positive indication of both satisfactory and unsatisfactory quality to reduce the uncertainty of the test results, and to provide such a device that is easy to use encourage frequent use. It is also among the objects of the present invention to provide such a device which uses a probe having terminals the switch in polarity to avoid ionization of the fluid between the terminals.

The fluid testing device of the present invention is adapted to measure the quality of fluid by measuring its conductivity. The conductivity of the fluid increases as the content of contaminants, for example water, increases. Generally, the testing device of the present invention comprises measuring means for measuring the conductivity of the fluid, and indicating means responsive to the measuring means for indicating the quality of the fluid based upon its measured conductivity.

The indicating means preferably comprises a plurality of actuable signal devices which, when actuated, indicate the quality of the fluid and means for actuating an increasing number of the actuable signal devices as the measured conductivity reaches predetermined values corresponding to predetermined moisture contents. There are preferably four such signal devices, for example, light emitting diodes. The first of these diodes is illuminated at a nominal conductivity level to provide a positive indication that the device is operational and that fluid quality is satisfactory. The other diodes are lit for example at conductivities corresponding to 1 percent, two percent, and three percent moisture content in the fluid. The device may include a probe having terminals of alternating polarity for contacting the fluid being measured.

The device thus provides a way of testing the quality of fluid by measuring its conductivity and comparing it to preselected bench marks. The device is of simple and inexpensive construction, and can be made to be hand held for easy use. The device provides a positive indication of both satisfactory and unsatisfactory quality to reduce the uncertainty of the test results, avoids ionization problems and does not require special training or instruction to use it.

## BRIEF DESCRIPTION OF THE DRAWINGS

Figure 1 is a plan view of a brake fluid testing device constructed according to the principles of this invention; and

Figure 2 is a schematic diagram of the electronic circuitry incorporated into one preferred embodiment of the fluid testing device.

Figure 3 is a schematic diagram of the electronic circuitry incorporated into another preferred embodiment of the fluid testing device.

Corresponding reference characters indicate corresponding parts throughout the several views of the drawings.

## DETAILED DESCRIPTION OF THE PREFERRED EMBODIMENT

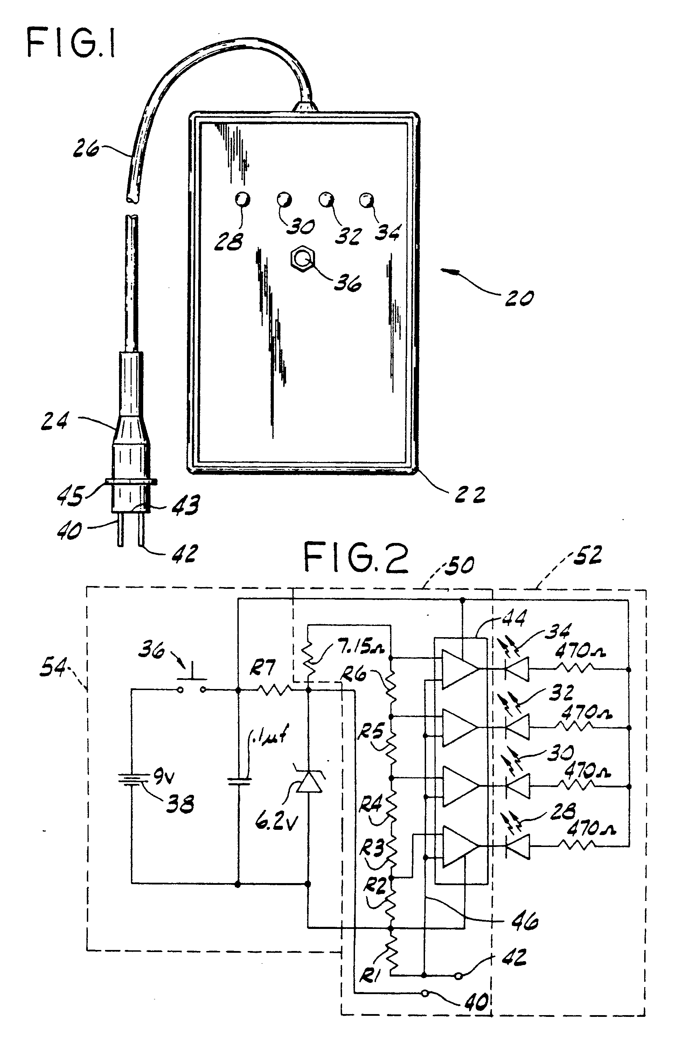

A brake/coolant fluid testing device constructed according to the principles of this invention is indicated generally as 20 in Figure 1. The device is adapted to test the quality of brake/coolant fluid by measuring its conductivity or resistance. As used throughout conductivity and resistance are used interchangably. In fact, the preferred embodiment of this invention described herein measures the conductivity of brake/coolant fluid by comparing it to reference resistances. The device 20 comprises a case 22 and a test probe 24 connected to the case 22 with a coaxial cord 26.

The case 22 has four actuable signal devices, namely light emitting diodes 28, 30, 32 and 34. In this preferred embodiment diode 28 is green, diode 30 is amber, diode 32 is orange, and diode 34 is red, although the diodes could be some other color or the diodes could all be the same color. The case also has a push button momentary switch 36 for actuating the testing device, as described below.

The case 22 contains a circuit on a printed circuit board, which is shown schematically in Figure 2. The case 22 also includes a compartment (not shown) for containing a battery 38 for powering the testing device. This battery 38 is preferably a standard 9v battery.

The test probe 24 comprises two terminals or contacts 40 and 42, electrically separated from each other by an insulator 43. The circuit 38 is preferably tuned to the size and shape of the contacts 40 and 42, which affects the conductivity measured between the contacts. One of the two leads of coaxial cord 26 connects each of the contacts 40 and 42 to the circuit. A flange 45 may be provided on the probe 24 to indicate the level to which the probe 24 should be immersed in the fluid to be tested.

The circuit compares the conductivity (or resistance) between the contacts 40 and 42 with predetermined bench mark conductivities established by resistors R1-R6 in the circuit. These bench mark conductivities have been empirically determined to correspond with preselected moisture content levels. In this preferred embodiment the conductivities correspond to the conductivity of an average brake fluid with just slightly greater than about 0% moisture; the conductivity of an average brake fluid with about 1% moisture; the conductivity of an average brake fluid with about 2% moisture; and the conductivity of an average brake fluid with about 3% moisture. The inventor has empirically determined that for most brands of brake fluid, the bench mark values of conductivity and its equivalent resistance are as follows:

TABLE I

| BRAKE FLUID | | |
|---|---|---|
| CONDUCTIVITY | RESISTANCE | MOISTURE CONTENT |
| $2.8 \times 10^{-6}$ mho | 350. kohms | 0 + % (Green) |
| $3.01 \times 10^{-5}$ mho | 33.2 kohms | ~1% (Yellow) |
| $3.39 \times 10^{-5}$ mho | 29.5 kohms | ~2% (Orange) |
| $3.94 \times 10^{-5}$ mho | 25.4 kohms | ~3% (Red) |

3

Of course the measured conductivity between the contacts 40 and 42 depends on the size, shape and spacing of the terminals on the probe. With a different probe the measured conductivity may vary somewhat, but the device is easily calibrated by adjusting the resistances in the circuit to correspond with the measured resistances between the contacts for selected moisture levels. The levels stated in the above Table I for brake fluid testing are based on calibration resistors R1 and R7 having values of 16.2 kohms and 1 kohms, respectively, and resistors R2-6 having resistances of 1 kohms, 1.5 kohms, 1 kohms, 142 ohms and 255 ohms, respectively. Similar values are applicable to other fluids to be tested.

If the coolant fluid to be tested is ethylene glycol, the conductivity levels may be as follows:

TABLE II

| COOLANT | | |
|---|---|---|
| CONDUCTIVITY | RESISTANCE | INDICATION |
| $1.30 \times 10^{-3}$ mho | 770 ohms | Green |
| $1.55 \times 10^{-3}$ mho | 647 ohms | Yellow |
| $1.92 \times 10^{-3}$ mho | 520 ohms | Orange |
| $2.41 \times 10^{-3}$ mho | 415 ohms | Red |

The levels stated in the above Table II for coolant testing are based on calibration resistors R1 and R7 having values of 510 kohms and 330 ohms, respectively, and resistors R2-R6 having resistances of 464 ohms, 1.82 kohms, 1 kohms, 309 ohms and 309 ohms, respectively. Evaluating the quality of coolant presents a slightly different problem than the evaluation of brake fluid although the same apparatus can be used. When testing brake fluid, the concern is the amount of free water in the fluid which can be detected by measuring conductivity. When testing coolant, the concern is the amount of minerals or ions in the coolant which can also be detected by measuring conductivity. For coolant, the conductivity levels depend, in part, on the ion levels considered to be unacceptable.

Depending on the type of automotive cooling system, the coolant may range from 100% ethylene glycol to a 50-50 mix of ethylene glycol and water. Preferably, the water is distilled so that no minerals or ions are present in the mixture. As the coolant is used and moves through the cooling system, the motion generates charged particles within the coolant increasing the ion content of the coolant which, in turn, increases the conductivity. Minerals and ions from the metals in contact with the coolant begin to dissolve in the coolant thereby making the coolant more acidic and increasing its conductivity. In places where coolant becomes trapped, such as in a liner or O-ring seal, the trapped coolant acts as an electrolytic cell, similar to a battery, and eats away at the metal, rubber and plastic parts of the cooling system. This action is similar to the discharging of a battery in which lead cells are removed from the battery plates during the electrolytic process. Instead, iron ions from the cast iron block or aluminum ions from the aluminum head dissolve in the coolant. In the past, corrosion and rust inhibitors have been added to the coolant to reduce its acidity. However, these inhibitors do not prevent electrolysis because the ions are still present in the coolant. The invention permits testing of the coolant to determine its conductivity which indicates its ion content and, therefore, its quality.

Reference character 44 illustrates an integrated circuit (IC) comprising a plurality of voltage comparators (four are illustrated) for comparing the resistance across terminals 40 and 42 to the resistors R1-R6. For example, IC 44 may be an SM8912 or LM399N manufactured by Motorola, National Semiconductor, or Texas Instruments. As the resistance across terminals 40 and 42 decreases, the voltage applied to line 46 changes to trip one or more of the comparators which, in turn, energizes its associated LED.

In the preferred embodiment of Figure 2 for use as a brake fluid tester, when the measured resistance between the contacts 40 and 42 is more than about 350 kohms and less than about 33.2 kohms, the circuit causes the diode 28 (the green diode) to light up. This provides a positive indication that the device is operational and that the quality of the brake fluid is satisfactory. When the measured resistance is more than about 33.2 kohms and less than about 29.5 kohms, the circuit causes both the diode 28 and the diode 30 (the amber diode) to light up indicating a somewhat reduced quality. When the measured resistance is more than about 29.5 kohm and less than about 25.4 kohms, the circuit causes the diodes 28, 30, and 32 (the orange diode) to light up indicating a more reduced quality. When the measured resistance is no more than about 25.4 kohms, the circuit causes all the diodes 28, 30, 32, and 34 (the red diode) to light up indicating a further reduced quality.

Thus, the device provides different, readily understood signals as the moisture content of the brake fluid increases. Resistors R1-R7 and IC 44 constitute measuring means 50 for measuring the conductivity of the brake fluid. Diodes 28-34 constitute indicating means 52 responsive to the measuring means 50 for indicating the quality of the brake fluid based upon its measured conductivity. Reference character 54 generally refers to the power supply for the device including a zener diode such as IN5234 for the brake fluid tester or IN5235 for the coolant tester.

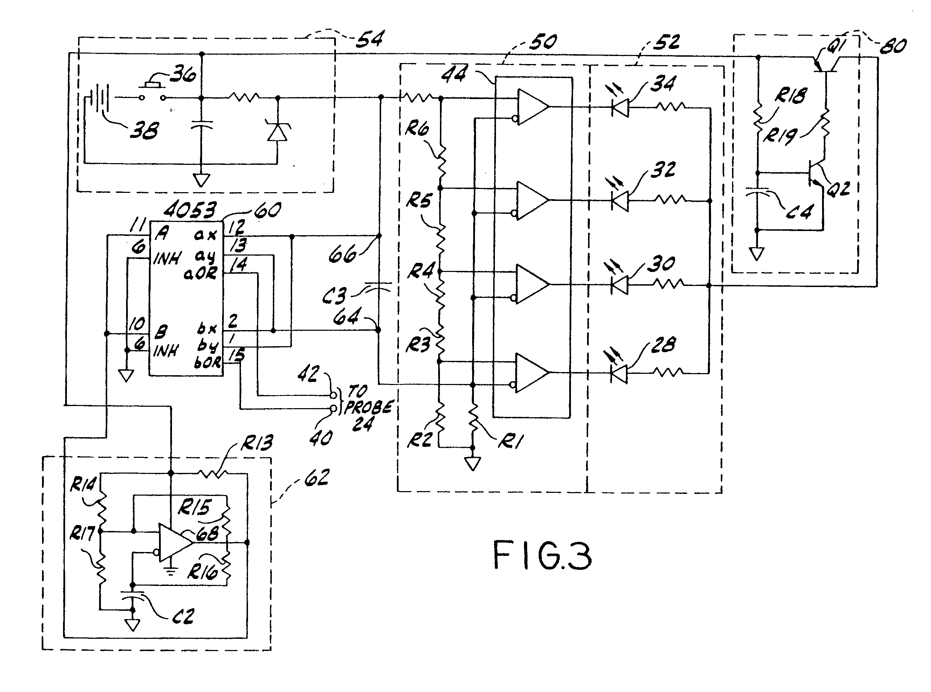

Referring to Figure 3, another preferred embodiment of the invention is illustrated in schematic form. For testing of certain fluids, ionization of the fluid between terminals 40 and 42 can occur which may affect the accuracy of the device. In order to avoid this, the polarity of the probes is alternated. This is accomplished by using an analog switch 60 between the measuring means 50 and the probe 24. A pulse generator 62 generates a square wave applied to inputs A and B of switch to selectively connect terminal 40 (pin 15) to either point 64 via pin 2 or point 66 via pin 1 and to selectively connect terminal 42 (pin 14) to either point 66 via pin 12 or point 64 via pin 13. Capacitor C2 controls the repetition rate of the square wave which is preferably 138 hertz. Capacitor C2 (0.1 microfarads) is successively charged and discharged via resistive network R13, R14, and RI7 (470 ohms, 100 kohms, and 100 kohms, respectively). This varying charge is applied to the inverting input of comparator 68 (LM339N) causing the output of comparator 68 to vary from high to low. Feedback resistors R15 and R16 (100 kohms and 10 ohms, respectively) maintain the state of comparator 68 during its charging and discharging. The output of comparator 68 is connected to inputs A and B to successively change the state of switch 60.

Capacitor C3 averages oscillations of the signals on each terminal of the probe 24 to avoid sudden variations in the charge applied to the comparators of IC 44 due to the switching. Any sudden variations may cause blinking. Furthermore, a delay circuit 80 may be provided to inhibit illumination of the diodes 28-34 until capacitor C3 is charged to avoid blinking of the diodes during the charging period. Until a capacitor C4 (10 microfarads) is charged via resistor R18 (5.6 megaohms), transistor switch Q2 (2N3904) is off which results in transistor switch Q1 (2N3905) being off and preventing illumination of the diodes 28-34. When capacitor C4 is charged, the base of transistor Q2 goes high to turn it on and apply a signal via resistor R19 (10 kohms) to turn on transistor Q1 thereby closing the circuit connected to the diodes and permitting their illumination. Capacitor C4 and resistor R18 have a time constant which is long enough to permit capacitor C3 to fully charge.

OPERATION

In operation, the probe 26 is immersed into the fluid to be tested, so that the contacts 40 and 42 are completely immersed in the fluid. For example, the probe 26 can be inserted into the master cylinder of the brake system, if it is being used to test brake fluid in an operating brake system, or it can be inserted into the coolant reservoir of the cooling system, if it is being used to test coolant in an operating coolant system, or it can be inserted into a container of brake fluid or coolant, if it is being used to test the condition of fluid before it is put in a system.

The push button 36 is pressed and the circuit causes the appropriate number of diodes to light up. If the conductivity is extremely low, corresponding to a very low moisture or ion content, only the green diode 28 lights up. This serves as a positive indication that the device is operational, and confirms that the quality of the fluid is satisfactory. For brake fluid, if the moisture content is about 1%, diodes 28 and 30 light up; if the moisture content is about 2%, diodes 28, 30, and 32 light up; and if the moisture content is at least about 3%, all of the diodes 28, 30, 32, and 34 light up. The increasing number of diodes gives a readily understood indication of the condition of the fluid, i.e., the moisture content for brake fluid and thus its boiling point or the ion content for coolant and thus its level of contamination. The more diodes that are lit, the more contaminated the fluid is, and thus the lower its quality. Furthermore, the color coding of the diodes further reinforces the meaning, the single green diode indicating satisfactory quality and the diodes ranging from amber, orange, to red representing deteriorating quality.

In view of the above, it will be seen that the several objects of the invention are achieved and other advantageous results attained.

As various changes could be made in the above constructions without departing from the scope of the invention, it is intended that all matter contained in the above description or shown in the accompanying drawings shall be interpreted as illustrative and not in a limiting sense.

## Claims

1. A fluid tester for measuring the quality of fluid, the device comprising:
a plurality of actuable signal devices which, when actuated, indicate the quality of fluid; and
measuring means for measuring the conductivity of the fluid and actuating the actuable signal devices in response to the comparison measurement.

2. The device according to claim 1 wherein tester is adapted to measure the quality of brake fluid and wherein the measuring means compares the conductivity of the brake fluid to a reference.

3. The device according to claim 1 wherein the tester is adapted to measure the quality of coolant and wherein the measuring means compares the conductivity of the coolant to a reference.

4. The device according to claim 1 wherein the measuring means comprises a probe having first and second terminals adapted to be in contact with the fluid, and means, connected to the probe, for comparing the conductivity of the fluid between the first and second terminals to a reference.

5. The device according to claim 4 wherein the measuring means further comprises means for switching the polarity of the first and second terminals and means for repetitively actuating the switching means whereby the first and second terminals successively change polarity to inhibit ionization of the fluid between the first and second terminals.

6. The device according to claim 1 wherein the measuring means further comprises a probe having first and second terminals adapted to be in contact with the fluid, means for selectively connecting the first or second terminals to the comparing means and means for repetitively actuating the connecting means whereby successive selective connecting of the first and second terminals to the comparing means inhibits ionization of the fluid between the first and second terminals.

7. The device according to claim 6 wherein the measuring means further comprises means, connected to the probe, for comparing the conductivity of the fluid between the first and second terminals to a plurality of references and a capacitor connected between the first and second terminals, and further comprising means for inhibiting the indicating means during the initial charging period of the capacitor.

8. The device according to claim 4 wherein the comparing means for comparing the conductivity of the fluid to a reference comprises means for comparing the resistance of the fluid to a plurality of reference resistances.

9. The device according to claim 1 further comprises means for actuating an increasing number of the actuable signal devices as the measured conductivity of the fluid increases.

10. The device according to claim 9 where the means for actuating the signal devices actuates at least one actuable signal device when the measured conductivity is nominal, to indicate that the device is operational.

11. The device according to claim 1 wherein the measuring means actuates the actuable signal devices according to preselected bench mark values of the measured conductivity of the fluid.

12. The device according to claim 11 wherein the bench mark values are selected to correspond to empirically determined conductivities of fluid with preselected contamination contents.

13. The device according to claim 2 wherein there are at least four actuable signal devices, and wherein the means for comparing and actuating the signal devices comprises means for actuating a first one of the signal devices when conductivity corresponding to nominal moisture in the fluid is measured; means for actuating a second of the signal devices when conductivity corresponding to at least 1% moisture in the fluid is measured; means for actuating a third of the signal devices when conductivity corresponding to at least 2% moisture in the fluid is measured; means for actuating a fourth of the signal devices when conductivity corresponding to at least 3% of the moisture level in the fluid is measured.

14. The device according to claim 3 wherein there are at least four actuable signal devices, and wherein the means for comparing and actuating the signal devices comprises means for actuating a first of the signal devices when the measured conductivity corresponds to distilled water; means for actuating a second of the signal devices when the measured conductivity corresponds to a first level of ion content having a conductivity greater than distilled water; means for actuating a third of the signal devices when the measured conductivity corresponds to a second level of ion content greater than the first level; and means for actuating a fourth of the signal devices when the measured conductivity corresponds to a third level of ion content greater than the second level.

15. The device according to claim 9, 13, and 14, wherein the actuable signal devices comprises light emitting diodes.

16. A fluid testing device far measuring the quality of fluid, the device comprising:
a probe having first and second terminals adapted to be in contact with the fluid;
measuring means, connected to the probe, for measuring a parameter of the fluid between the first and second terminals representative of the quality of the fluid;

means for switching the polarity of the first and second terminals;

means for repetitively actuating the switching means whereby the first and second terminals successively change polarity to inhibit ionization of the fluid between the first and second terminals; and

indicating means responsive to the measuring means for indicating the quality of the fluid based upon the measured parameter.

17. The device according to claim 16 wherein the parameter is conductivity; wherein the measuring means comprises means, connected to the probe, for comparing the conductivity of the fluid between the first and second terminals to a plurality of references and a capacitor connected between the first and second terminals; and further comprising means for inhibiting the indicating means during the initial charging period of the capacitor.

18. The device according to claim 6 and 17 wherein the switching means comprises an analog switch between the comparing means and the first and second terminals and a pulse generator for generating pulses for repetitively actuating the analog switch.

19. A method of testing coolant for measuring the quality of coolant, the method comprising the steps of:

measuring the conductivity of the coolant; and

indicating the quality of the coolant based upon its measured conductivity.

20. A method of testing brake fluid for measuring the quality of brake fluid, the method comprising the steps of:

measuring the conductivity of the brake fluid; and

indicating the quality of the brake fluid based upon its measured conductivity.

# FIG.1

# FIG.2

FIG.3